# EUROPEAN PATENT SPECIFICATION

(11) **EP 2 736 968 B1**
(45) Date of publication and mention of the grant of the patent: **11.03.2020**
(21) Application number: 12759250.9
(22) Date of filing: 27.07.2012
(51) Int. Cl.: C08J 11/10, B01J 19/12, C08L 67/00, C08L 77/00, C07C 51/09, C07C 63/26, B01J 19/20, C25B 15/08, C25B 1/34

(54) **METHOD AND APPARATUS FOR THE RECYCLING OF POLYMERIC MATERIALS VIA DEPOLYMERIZATION PROCESS**
VERFAHREN UND VORRICHTUNG ZUR WIEDERVERWERTUNG VON POLYMERMATERIALIEN IN EINEM DEPOLYMERISATIONSPROZESS
PROCÉDÉ ET APPAREIL UTILISABLES EN VUE DU RECYCLAGE DE MATÉRIAUX POLYMÈRES PAR UN PROCESSUS DE DÉPOLYMÉRISATION

(30) Priority: 27.07.2011 IT MI20111411
(43) Date of publication of application: 04.06.2014
(73) Proprietor: GR3N SAGL, 6963 Pregassona - Lugano (CH)
(72) Inventor: PARRAVICINI, Matteo, 22100 Como (IT); CRIPPA, Maurizio, 22032 Albese Con Cassano (CO) (IT); BERTELÈ, Matteo Vittorio, 22030 Montorfano (CO) (IT)
(74) Representative: Cernuzzi, Daniele
(86) International application number: PCT/IB2012/053867
(87) International publication number: WO 2013/014650

(56) References cited:
- EP-A1- 1 964 877
- WO-A1-2006/021063
- JP-A- 2003 292 594
- US-A1- 2005 096 482
- US-A1- 2007 102 848
- US-B1- 6 184 427

## Description

### TECHNICAL FIELD

The present invention concerns a method and an apparatus for the recycling of polymeric materials, specifically polyesters and polyamides, via depolymerization process.

### BACKGROUND ART

As is known, various polymeric materials, widely used for manufacturing a number of products, can be recovered and reused at the end of the product working life, instead of being sent to the landfill.

In particular, some polymeric materials, for example polyesters and polyamides, can be depolymerized, i.e. reduced to the form of monomers (or oligomers), and then re-polymerized to create new products.

In general, these materials are firstly ground and reduced to granules, after which they undergo depolymerization reactions in appropriate process conditions.

Although different depolymerization reactions are known, in particular of polyesters and polyamides, at the moment industrial processes do not appear to be available that are fully satisfactory, above all in terms of simplicity, yield, efficiency and process speed.

A reaction described at experimental or laboratory level is not always suitable for industrial exploitation in concrete terms; in certain cases, a given reaction can be exploited only by recourse to relatively complicated and/or costly plants and/or via processes which as a whole are not particularly advantageous, or are in any case susceptible of improvement.

For example, depolymerization reactions for PET (polyethylene terephthalate) are described, in general terms but without operating details that make it possible to advantageously implement them on an industrial scale, in the following publications.

The article "Microwave-assisted depolimerization of Poly(ethylene-terephtalate) PET at atmospheric pressure", published in "Advances in Polymer Technology", Vol. 25, No. 4, p. 242-246 (2006), proposes using a PET depolymerization reaction in the presence of alcohols (pentanol) which cannot be easily implemented in an industrial plant; the reaction is generically assisted by microwaves, but no details are provided on a possible industrial plant.

Another PET depolymerization reaction is described in "Alkali decomposition of Poly(ethylene-terephatalate) with sodium Hydroxide in nonaqueous ethylene glycol: A study on recycling of terephthalic acid and Ethylene glycol", Journal of Applied Polymer Science, 1997, Vol.63; No.5, 595-601; also this publication does not provide indications on the possible industrial exploitation of the reaction.

EP1964877 ("Method of depolymerizing polyester and unsaturated polyester and method of recovering polyester monomer with the depolymerization method") describes a depolymerization reaction for polyesters based on a basic hydrolysis, assisted by microwaves; no construction details of an industrial plant or operating parameters of an industrial process are described.

EP2176327 ("Method for the chemical depolymerization of waste polyethylene terepthalate") describes a process in two stages (melting with catalysts which activate the reaction and subsequent depolymerization) which requires the use of catalysts.

The article "Rapid microwave induced depolymerization of polyamide-6", Polymer 41 (2000), 4361-4365 describes a depolymerization reaction based on a water/H3PO4 mixture which involves an incomplete reaction, with the presence of oligomers.

### DISCLOSURE OF INVENTION

An object of the present invention is to provide an apparatus and a method for recycling polyesters and polyamides via depolymerization which is free from the drawbacks of the known art highlighted here; in particular, an object of the invention is to provide an apparatus and a method which allow recovery of said materials on an industrial scale in a simple, effective and inexpensive manner.

According to said objects, the present invention relates to a method and an apparatus for recycling polymeric materials, specifically polyesters and polyamides, via depolymerization as defined in essential terms in the attached claims 1 and 8 respectively and, for the preferred additional characteristics, in the dependent claims.

The method and the apparatus according to the invention allow effective recovery of the monomers constituting the polymers treated (polyesters and polyamides) and re-introduction of the corresponding polymers into the production cycle, also on an industrial scale, in a simple, effective and inexpensive manner.

A microwave depolymerization reactor operating continuously is used, having a particular configuration which allows optimal performance of the depolymerization reactions.

Further advantages of the invention are the following:
- the reactor operates continuously, consequently eliminating the dead time typical of discontinuous processes;
- the division of the apparatus into two functional units allows the creation of a versatile overall system, as the first unit allows depolymerization of different polymers, with the same general configuration; the second unit can be modified according to the material treated;
- the method of the invention is substantially self-consistent in terms of reactants used, so that there is no reaction waste apart from the polymer contaminants;
- the method does not require melting of the polymers to be recycled before depolymerization;
- the depolymerization reaction performed by means of microwaves, thanks to the efficiency of the reaction, allows reduction of the energy costs with respect to the same process performed via other methods, for example by glycolysis, basic/acid transesterification, or methanolysis, using traditional heating.

### BRIEF DESCRIPTION OF THE DRAWINGS

Further characteristics and advantages of the present invention will appear clear from the description of the following non-limiting implementation examples, with reference to the accompanying figures in which:
- figure 1 is the diagram of an apparatus for recycling of polymeric materials by means of depolymerization in accordance with the invention;
- figure 2 is a schematic view of a part of the apparatus of figure 1, in particular of a depolymerization reactor with associated reactant movement system;
- figure 3 shows schematically a possible configuration of a plurality of depolymerization reactors of the type shown in figure 2;
- figures 4-6 show schematically a detail of the reactor of figure 2, in particular a metering valve, in different operating positions.

### BEST MODE FOR CARRYING OUT THE INVENTION

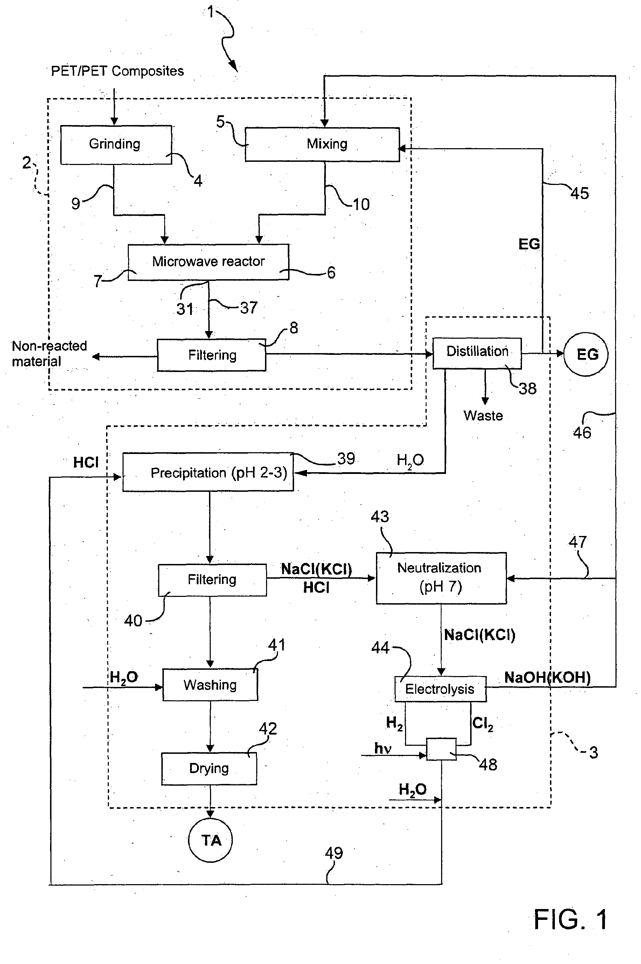

In figure 1, the number 1 indicates as a whole an industrial apparatus operating continuously for recycling of polymeric materials, in particular polyesters (and specifically polyethylene terephthalate and its isomers, PET) or polyamides (nylon 6, nylon 6,6), by depolymerization.

The apparatus 1 comprises two functional units 2, 3: a depolymerization unit 2 and a treatment unit 3.

The depolymerization unit 2 comprises a grinding unit 4 for preliminary mechanical treatment of the materials to be recycled, a mixing unit 5 in which a solvolytic mixture is prepared, at least one microwave depolymerization reactor 6 provided with a system 7 for movement of the reactants which allows the reactor 6 to operate continuously, and a filtering unit 8 acting on the reaction products coming out of the reactor 6.

In the grinding unit 4 the materials to be treated, for example polymeric waste materials, pure or coupled with other materials that cannot be separated from them by mechanical action, undergo mechanical grinding treatment which reduces the material into granules, in order to increase the surface area of the material and therefore the contact surface with the solvolytic mixture.

In general, the materials treated by the apparatus 1 are PET or other polyesters, or polyamides, or composite materials containing said polymers. In the following description particular reference will be made, by way of example, to the treatment of PET or composites containing PET.

The solvolytic mixture is prepared in the mixing unit 5; if the material to be recycled is PET or other polyester, or also polyamide, the solvolytic mixture consists for example of an alkaline hydroxide, such as NaOH, KOH, LiOH, in ethylene glycol (EG).

The ground material coming from.the grinding unit 4, constituting a solid phase, and the solvolytic mixture prepared in the mixing unit 5, constituting a liquid phase, are fed, via respective feed lines 9, 10, to the reactor 6, where they are mixed to form a heterogeneous reaction mixture and left to react.

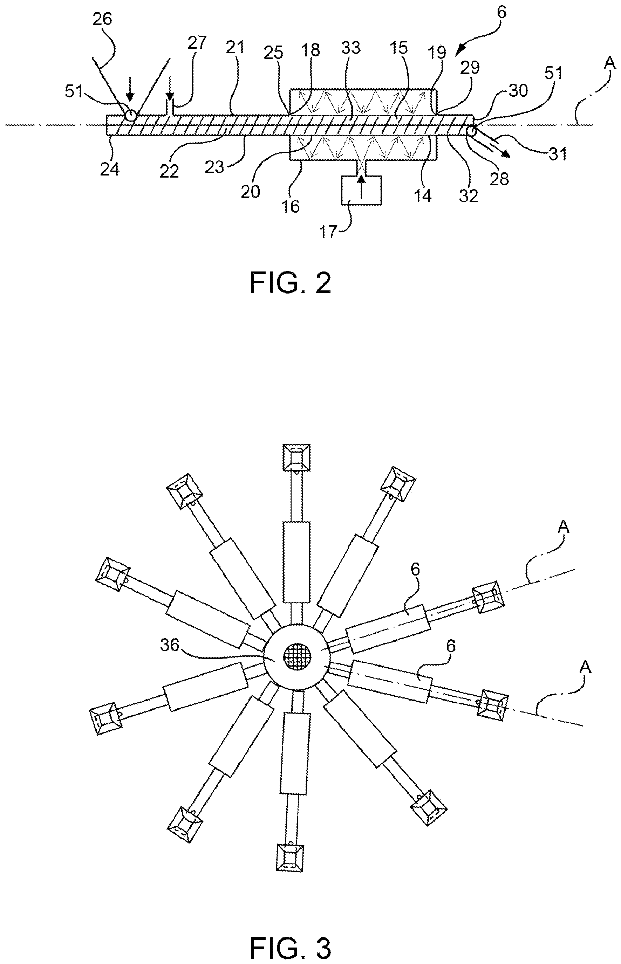

As illustrated in figure 2, the reactor 6 comprises a tubular casing 14, internally defining a reaction chamber 15 and housed inside a microwave chamber 16 provided with a microwave generator device.

The casing 14 extends substantially along and around a rectilinear axis A (for example, but not necessarily, substantially horizontal) between two opposite axial ends 18, 19 and has a lateral wall 20, for example substantially cylindrical with circular cross section, transparent to the microwave radiation and delimiting the reaction chamber 15.

For example, the lateral wall 20 is made of PTFE, or ceramic transparent to the microwave radiation, or glass covered internally by an anticorrosion coating transparent to the microwave radiation. Said anticorrosion coating can be produced for example with oxides, nitrides, carbides or silicides of metals, transition metals, lanthanides, actinides, in addition to mixed compounds; purely by way of example, the following can be used: TiSi2, TiO2, ZrC, ZrO2, ZrSi2, ZrN, Y2O3, SiC, BN.

The microwave chamber 16 is arranged coaxial and radially external to and around the casing 14, so as to surround the reaction chamber 15.

Microwaves with frequency between 300 MHz and 300 GHz and power density between 1 W/L and 1000 W/L are sent into the microwave chamber 16 via the device 17.

The movement system 7 is an Archimedean screw system which moves the reaction mixture (formed of material to be depolymerized and solvolytic mixture) substantially along the axis A (and in a spiral around the axis A) through the reactor 6 in a pre-determined direction of forward movement (in the example, from the end 18 towards the end 19).

In particular, the system 7 comprises a tubular feed duct 21, coaxial and substantially aligned with the casing 14 along the axis A, and an endless screw 22 housed inside the duct 21 and the casing 14.

The duct 21, delimited by a substantially cylindrical lateral wall 23 with circular section, is arranged upstream of the casing 14 in the direction of forward movement of the reaction mixture and extends along the axis A between two opposite longitudinal ends 24, 25.

The end 24 is provided with two separate inlets 26, 27 for the solid phase (polymeric material in granules) and the liquid phase (solvolytic mixture), which both flow into the duct 21 and mix with each other to form the reaction mixture; the opposite end 25 is connected to the end 18 of the casing 14 and communicates with the reaction chamber 15. In figure 2, the inlet 26 for the solid phase is positioned upstream (before) the inlet 27 for the liquid phase along the duct 21, but the position of the inlets 26, 27 and therefore the feed order of the two phases inside the duct 21 can be inverted with respect to the illustrative example.

Preferably, the side walls 20, 23 of the duct 21 and the casing 14 are aligned and have respective internal lateral surfaces which are continuous and flush with each other.

The movement system 7 also comprises a tubular discharge duct 28, coaxial and substantially aligned with the casing 14 along the axis A and arranged downstream of the casing 14 in the direction of forward movement.

Also the duct 28 extends along the axis A between two opposite longitudinal ends 29, 30: the end 29 is connected to the end 19 of the casing 14 and communicates with the reaction chamber 15, while the opposite end 30 is provided with an outlet 31, through which reaction products and non-reacted materials are discharged from the reactor 6.

Advantageously, also the discharge duct 28 is delimited by a substantially cylindrical lateral wall 32 with circular section, aligned with the lateral wall 20 of the casing 14 and having an internal lateral surface flush with the internal lateral surface of the casing 14 .

The feed duct 21, the casing 14 and the discharge duct 28 constitute a continuous channel 33 of which the reaction chamber 15 constitutes an intermediate portion.

The endless screw 22 extends along the axis A and rotates around the axis A, driven by a motor (known and not illustrated). The endless screw 22 extends substantially along the entire channel 33, and therefore through the feed duct 21, the casing 14 and the discharge duct 28.

The endless screw 22 is preferably made of a metallic material and can be coated in a material with low dielectric permittivity, for example PTFE or a ceramic material.

The rotation speed of the endless screw 22 is established according to the quantity and dimensions of the material to be depolymerized, the length of the reaction chamber 15 and the microwave chamber 16, the concentration and nature of the solvolytic mixture, the required contact time between the reactants and the microwaves, and the diameter of the reactor 6.

The depolymerization reaction takes place in the reaction chamber 15 by means of the solvolytic mixture already described and promoted/assisted by the microwaves.

In the case of PET, for example, the reaction with the solvolytic mixture of NaOH (or KOH or other alkaline hydroxide) in ethylene glycol produces a solution of terephthalic acid (TA) and/or its salts (for example, sodic or potassic salt) in ethylene glycol.

Advantageously, the reaction temperature is between approximately 150 and approximately 350°C and the pressure between approximately 1 atm and approximately 20 atm.

The same solvolytic mixture just described for the PET can also be used for the depolymerization of polyamide, performed in analogous conditions; alternatively, for the polyamide the reaction is carried out in acid conditions, using an acid solvolytic mixture formed for example from mineral acids such as H3PO4, HCl, H2SO4, in water, mono and/or polyhydroxy alcohols, ethylene glycol.

According to a preferred embodiment of the invention (but not necessarily), the apparatus 1 comprises a plurality of reactors 6 of the type just described, operating in parallel. For example, as illustrated schematically in figure 3, the reactors 6 are arranged radially around a common header 36; in particular, the reactors 6 extend along respective axes A which depart radially from the header 36, spaced at an angle from one another.

The header 36 collects the non-reacted materials and the reaction products coming from the various reactors 6 to convey them to the filtering unit 8 and then to the subsequent treatment unit 3.

With reference again to figure 1, the outlet 31 of the reactor 6 is connected, via an outlet line 37, to the filtering unit 8, configured so as to separate the reaction products (in general, monomers and any oligomers) to be recovered, which are sent to the treatment unit 3, from the non-reacted materials.

The treatment unit 3 is positioned upstream of the reactor 6 and filtering unit 8 and serves to perform recovery and purification operations, and any reconversion of the reaction products and by-products, such as for example filtering, precipitation, neutralization, centrifugation, dialyzation, distillation, electrolysis and photochemical reactions.

For example, in the embodiment of figure 1 the treatment unit 3 comprises: a distillation unit 38, a precipitation unit 39, a filtering and/or centrifugation unit 40, a washing unit 41, a drying unit 42, a neutralization unit 43 and an electrolysis unit 44.

The distillation unit 38 is fed with the reaction products coming from the filtering unit 8 (comprising mainly a solution of terephthalic acid and/or its salts in ethylene glycol) and produces ethylene glycol, a solution of terephthalic acid and/or relative salts, and waste products; the filtering unit 8 is connected by means of a recirculation line 45 to the mixing unit 5, in order to re-use, at least partly, the ethylene glycol as a solvent in the solvolytic mixture.

The precipitation unit 39 receives a solution containing terephthalic acid to be recovered, obtained from the distillation unit 38, to which water is added. The solution is treated in the precipitation unit 39 with mineral acids (for example, HCl) until achieving a pH of approximately 2÷3, thus obtaining precipitation of the terephthalic acid; the terephthalic acid is removed, in the filtering and/or centrifugation unit 40, from the acid aqueous solution and then sent to the washing unit 41 and to the drying unit 42. Terephthalic acid is obtained with a purity level sufficient to undergo a new polymerization reaction.

The aqueous acid solution resulting from the removal of the terephthalic acid is neutralized in the neutralization unit 43, for example with alkaline bases such as NaOH or KOH, up to neutral pH; the resulting salts, NaCl or KCl, are used in the electrolysis unit 44 for an electrolytic chloralkali process.

At the outlet of the electrolysis unit 44, NaOH or KOH are obtained and, separately, chlorine and hydrogen; NaOH or KOH are sent, via respective recirculation lines 46, 47, partly to the mixing unit 5, to be re-used for preparation of the solvolytic mixture, and partly to the neutralization unit 43; chlorine and hydrogen are collected in a reaction chamber 48 where they form HCl which is recirculated, by means of a recirculation line 49, to the precipitation unit 39 to be re-used in the precipitation reaction of the terephthalic acid from the corresponding salt.

The same operations are substantially performed also in the case of the treatment of polyamides.

According to one aspect of the invention, the reactor 6 is provided with two metering valves 51 (only schematically indicated in figure 2), positioned upstream and downstream respectively of the reaction chamber 15, on the inlet 26 and on the outlet 31. The valves 51 allow the passage of material from the outside to the inside of the reactor 6 (inlet valve 51, positioned at the inlet 26) and vice versa from the inside to the outside of the reactor 6 (outlet valve 51, located at the outlet 31). The valves 51 are configured so as to transfer material from the outside into the reactor 6 or from the reactor 6 to the outside without the two environments (inside and outside) ever being in direct communication and therefore ensuring that the reaction chamber 15 remains at controlled pressure.

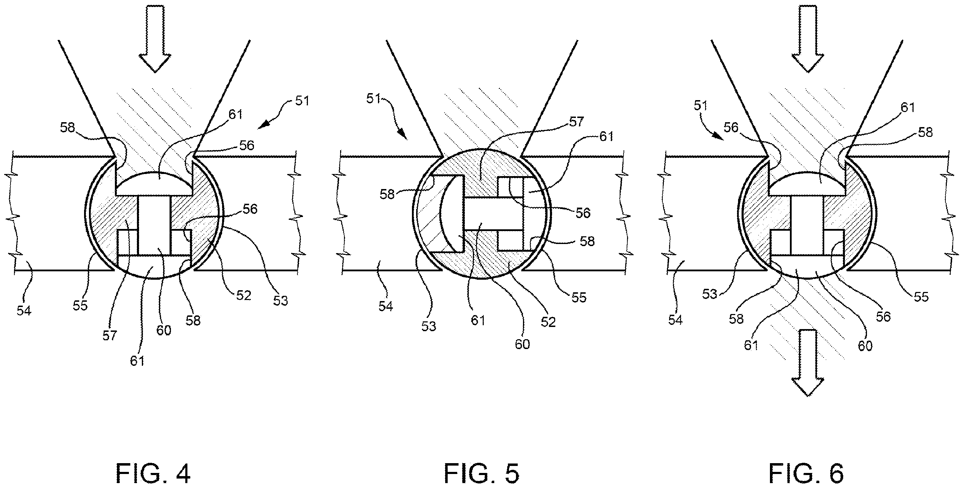

In particular, as shown in detail in figures 4-6, each valve 51 comprises a revolving valve body 52, having for example a substantially cylindrical or spherical shape and rotating around a rotation axis inside a seat 53, formed in a fixed casing 54 and delimited by a wall 55.

The valve body 52 presents two opposite cavities 56, divided by a partition 57 and having respective diametrically opposite access apertures 58 formed in the wall 55, and a moving piston 60, for example arranged across the partition 57 and sliding along an axis perpendicular to the axis of rotation and controlled from the outside according to the position; the piston 60 has two axially opposite heads 61, housed in respective cavities 56; each head 61 is shaped so as to close a respective aperture 58 and therefore a cavity 56 and moves between a retracted position, in which the head 61 is positioned on the bottom of the cavity 56, and an extended position, in which the head 61 occupies the cavity 56 and closes the aperture 58; when a head 61 is in the extended position, the opposite head 61 is in the retracted position and vice versa. Each cavity 56 is alternately filled with material to be transferred (inside or outside the reactor 6), when the respective head 61 of the moving piston 60 is in the retracted position, and with the same head 60 which is brought to the extended position. During rotation of the valve body 52 in the seat 53, the valve body 52 and the head 60 which is in the extended position are substantially in contact with the wall 55 and slide against the wall 55.

## Claims

1. A method for the recycling of polyesters and/or polyamides via depolymerization process, comprising a depolymerization reaction of the material to treat with a solvolytic mixture; the method being **characterized in that** the depolymerization reaction comprises the steps of: providing a microwave depolymerization reactor (6) extending substantially along an axis (A); feeding the material to treat and the solvolytic mixture, defining the reactants of the depolymerization reaction and constituting a solid phase and a liquid phase respectively, to the reactor (6) via respective feed lines (9, 10); moving the reactants of the depolymerization reaction through the reactor (6) via an Archimedean screw moving system (7) that moves the reactants substantially along the axis (A) and mixes the reactants with each other to form a heterogeneous reaction mixture; radiating the reactants, while passing through a reaction chamber (15) of the reactor (6), with microwave radiation to carry out the depolymerization reaction; the reaction chamber (15) being defined inside a tubular casing (14) of the reactor (6) having a lateral wall (20) transparent to microwave radiation and housed inside a microwave chamber (16), which is arranged coaxial and radially external to and around the casing (14) so as to surround the reaction chamber (15); the reactants being moved by means of an endless screw (22) that extends along the axis (A) through the reaction chamber (15) and rotates about the axis (A).

2. A method according to claim 1, wherein the endless screw (22) is made of metallic material and is coated with a low dielectric permittivity material or PTFE or ceramic material.

3. A method according to claim 1 or 2, wherein the lateral wall (20) of the casing (14) is made of PTFE, or ceramic material transparent to microwave radiation, or glass internally coated by an anticorrosive coating transparent to microwave radiation.

4. A method according to claim 3, wherein the anticorrosive coating is made of oxides, nitrides, carbides or silicides of metals, transition metals, lanthanides, actinides or mixed compounds thereof.

5. A method according to one of the preceding claims, wherein the solvolytic mixture comprises ethylene glycol and at least one alkaline hydroxide, for example NaOH, KOH, LiOH.

6. A method according to one of the preceding claims, comprising an electrolytic chloralkali process for recovering caustic soda or caustic potash.

7. A method according to one of the preceding claims, comprising the steps of feeding the reactor (6) with polymeric material to be treated and removing from the reactor (6) reaction products and non-reacted materials, by means of respective metering valves (5) in order to transfer material from the outside into the reactor (6) and from the reactor (6) to the outside without the inside and the outside of the reactor (6) ever being in direct communication.

8. An apparatus (1) for the recycling of polyesters and/or polyamides via depolymerization process, comprising a depolymerization unit (2) having at least one depolymerization reactor (6), in which a depolymerization reaction of the material to treat with a solvolytic mixture occurs, and a treatment unit (3) for treating products coming out of the depolymerization unit (2); the apparatus comprising respective feed lines (9, 10) connected to the reactor (6) for feeding the reactor (6) with the material to treat and the solvolytic mixture, defining the reactants of the depolymerization reaction and constituting a solid phase and a liquid phase respectively; the apparatus (1) being **characterized in that** the reactor (6) is a microwave depolymerization reactor that extends substantially along an axis (A) and is provided with a moving system (7) of the reactants that allows the reactor (6) to operate continuously; the moving system (7) being an Archimedean screw system that moves the reactants substantially along the axis (A) through the reactor (6) and mixes the reactants to form a heterogeneous reaction mixture; the reactor (6) comprising a tubular casing (14), which extends along and around the axis (A) and has a lateral wall (20) transparent to microwave radiation and defining internally a reaction chamber (15); the casing (14) being housed inside a microwave chamber (16) which is arranged coaxial and radially external to and around the casing (14), so as to surround the reaction chamber (15), and being provided with a microwave generator device (17); the moving system (7) comprising a tubular supply conduit (21), coaxial and substantially aligned with the casing (14) along the axis (A), and an endless screw (22) which extends along the axis (A) inside the supply conduit (21) and the casing (14) and rotates around the axis (A).

9. An apparatus according to claim 8, wherein the endless screw (22) is made of metallic material and is coated with a low dielectric permittivity material or PTFE or ceramic material.

10. An apparatus according to claim 8 or 9, wherein the lateral wall (20) of the casing (14) is made of PTFE, or ceramic material transparent to microwave radiation, or glass internally coated by an anticorrosive coating transparent to microwave radiation.

11. An apparatus according to claim 10, wherein the anticorrosive coating is made of oxides, nitrides, carbides or silicides of metals, transition metals, lanthanides, actinides or mixed compounds thereof.

12. An apparatus according to one of the claims 8 to 11, wherein the solvolytic mixture comprises ethylene glycol and at least one alkaline hydroxide, for example NaOH, KOH, LiOH.

13. An apparatus according to one of the claims 8 to 12, and comprising a plurality of reactors (6) operating in parallel and arranged radially around a common header (36).

14. An apparatus according to one of the claims 8 to 13, wherein the treatment unit (3) comprises an electrolysis unit (44) for performing an electrolytic chloralkali process for recovering caustic soda or caustic potash.

15. Apparatus as claimed in one of the claims 8 to 14, comprising two metering valves (51), positioned upstream and downstream of the reaction chamber (15) and configured in order to transfer material from the outside into the reactor (6) or from the reactor (6) to the outside without the inside and the outside of the reactor (6) ever being in direct communication.

## Patentansprüche

1. Verfahren zur Wiederverwertung von Polyestern und/oder Polyamiden durch ein Depolymerisationsverfahren, umfassend eine Depolymerisationsreaktion des Materials zum Behandeln mit einer solvolytischen Mischung; wobei das Verfahren **dadurch gekennzeichnet ist, dass** die Depolymerisationsreaktion die Schritte umfasst des:
Bereitstellens eines Mikrowellen-Depolymerisationsreaktors (6), der sich im Wesentlichen auf einer Achse (A) entlang erstreckt;
Zuführen des Materials einer Behandlung und der solvolytischen Mischung, die die Reaktanden der Depolymerisationsreaktion definieren und jeweils eine feste Phase und eine flüssigen Phase bilden, dem Reaktor (6) durch jeweilige Zuführleitungen (9, 10);
Bewegen der Reaktanden der Depolymerisationsreaktion durch den Reaktor (6) über ein archimedisches Schneckenbewegungssystem (7), das die Reaktanden im Wesentlichen der Achse (A) entlang bewegt und die Reaktanden miteinander mischt, um eine heterogene Reaktionsmischung zu bilden; Bestrahlen der Reaktanden, während sie durch eine Reaktionskammer (15) des Reaktors (6) hindurchgehen, mit Mikrowellenstrahlung, um die Depolymerisationsreaktion auszuführen; wobei die Reaktionskammer (15) innerhalb eines röhrenförmigen Gehäuses (14) des Reaktors (6) definiert ist, das eine seitliche Wand (20) aufweist, die für Mikrowellenstrahlung transparent und innerhalb einer Mikrowellenkammer (16) untergebracht ist, die koaxial und radial extern von dem und um das Gehäuse (14) angeordnet ist, um die Reaktionskammer (15) zu umgeben; wobei die Reaktanden durch eine Endlosschnecke (22) bewegt werden, die sich der Achse (A) entlang durch die Reaktionskammer (15) erstreckt und um die Achse (A) rotiert.

2. Verfahren nach Anspruch 1, wobei die Endlosschnecke (22) aus metallischem Material hergestellt und mit einem Material geringer dieletrischer Permittivität oder PTFE oder Keramikmaterial beschichtet ist.

3. Verfahren nach Anspruch 1 oder 2, wobei die seitliche Wand (20) des Gehäuses (14) aus PTFE oder Keramikmaterial, das für Mikrowellenstrahlung transparent ist, oder Glas, das intern durch eine Antikorrosionsbeschichtung, die für Mikrowellenstrahlung transparent ist, beschichtet ist, hergestellt ist.

4. Verfahren nach Anspruch 3, wobei die Antikorrosionsbeschichtung aus Oxiden, Nitriden, Carbiden oder Siliciden von Metallen, Übergangsmetallen, Lanthaniden, Actiniden oder gemischten Verbindungen davon hergestellt ist.

5. Verfahren nach einem der vorhergehenden Ansprüche, wobei die solvolytische Mischung Ethylenglycol und mindestens ein alkalisches Hydroxid, beispielsweise NaOH, KOH, LiOH, umfasst.

6. Verfahren nach einem der vorhergehenden Ansprüche, umfassend ein elektrolytisches Chloralkaliverfahren zum Gewinnen von Ätznatron oder Kalilauge.

7. Verfahren nach einem der vorhergehenden Ansprüche, umfassend die Schritte des Beschickens des Reaktors (6) mit zu behandelndem polymerem Material und Entfernen, aus dem Reaktor (6), von Reaktionsprodukten und nichtreagierten Materialien durch jeweilige Dosierventile (5), um Material von der Außenseite in den Reaktor (6) und von dem Reaktor (6) zur Außenseite zu überführen, ohne dass die Innenseite und die Außenseite des Reaktors (6) sich je in direkter Kommunikation befinden.

8. Vorrichtung (1) zur Wiederverwertung von Polyestern und/oder Polyamiden durch ein Depolymerisationsverfahren, umfassend eine Depolymerisationseinheit (2), die mindestens einen Depolymerisationsreaktion (6) aufweist, wobei eine Depolymerisationsreaktion des Materials zum Behandeln mit einer solvolytischen Mischung erfolgt, und eine Behandlungseinheit (3) zum Behandeln von Produkten, die aus der Depolymerisationseinheit (2) kommen; wobei die Vorrichtung jeweilige Zuführleitungen (9, 10) umfasst, die an den Reaktor (6) angeschlossen sind, zum Beschicken des Reaktors (6) mit dem zu behandelnden Material und der solvolytischen Mischung, die die Reaktanden der Depolymerisationsreaktion definieren und jeweils eine feste Phase und eine flüssige Phase bilden; wobei die Vorrichtung (1) **dadurch gekennzeichnet ist, dass** der Reaktor (6) ein Mikrowellendepolymerisationsreaktor ist, der sich im Wesentlichen einer Achse (A) entlang erstreckt und mit einem Bewegungssystem (7) der Reaktanden ausgestattet ist, das dem Reaktor (6) gestattet, kontinuierlich zu arbeiten; wobei das Bewegungssystem (7) ein archimedisches Schneckensystem ist, das die Reaktanden im Wesentlichen der Achse (A) entlang durch den Reaktor (6) bewegt und die Reaktanden zum Bilden einer heterogenen Reaktionsmischung mischt; wobei der Reaktor (6) ein röhrenförmiges Gehäuse (14) umfasst, das sich der Achse (A) entlang und um diese herum erstreckt und eine seitliche Wand (20) aufweist, die für Mikrowellenstrahlung transparent ist und intern eine Reaktionskammer (15) definiert; wobei das Gehäuse (14) innerhalb einer Mikrowellenkammer (16) untergebracht ist, die koaxial und radial extern zu und um das Gehäuse (14) angeordnet ist, um die Reaktionskammer (15) zu umgeben und mit einer Mikrowellengeneratorvorrichtung (17) versehen ist; wobei das Bewegungssystem (7) eine röhrenförmige Belieferungsleitung (21), die koaxial zu und im Wesentlichen auf das Gehäuse (14) der Achse (A) entlang ausgerichtet ist, und eine Endlosschnecke (22) umfasst, die sich der Achse (A) entlang innerhalb der Belieferungsleitung (21) und des Gehäuses (14) erstreckt und um die Achse (A) rotiert.

9. Vorrichtung nach Anspruch 8, wobei die Endlosschnecke (22) aus metallischem Material hergestellt und mit einem Material geringer dielektrischer Permittivität oder PTFE oder Keramikmaterial beschichtet ist.

10. Vorrichtung nach Anspruch 8 oder 9, wobei die seitliche Wand (20) des Gehäuses (14) aus PTFE oder Keramikmaterial, das für Mikrowellenstrahlung transparent ist, oder Glas, das intern mit einer Antikorrosionsbeschichtung, die für Mikrowellenstrahlung transparent ist, beschichtet ist, hergestellt ist.

11. Vorrichtung nach Anspruch 10, wobei die Antikorrosionsbeschichtung aus Oxiden, Nitriden, Carbiden oder Siliciden von Metallen, Übergangsmetallen, Lanthaniden, Actiniden oder gemischten Verbindungen davon hergestellt ist.

12. Vorrichtung nach einem der Ansprüche 8 bis 11, wobei die solvolytische Mischung Ethylenglycol und mindestens ein alkalisches Hydroxid, beispielsweise NaOH, KOH, LiOH, umfasst.

13. Vorrichtung nach einem der Ansprüche 8 bis 12, und eine Mehrzahl von Reaktoren (6) umfassend, die parallel arbeiten und radial um einen gemeinsamen Rohrabzweiger (36) angeordnet sind.

14. Vorrichtung nach einem der Ansprüche 8 bis 13, wobei die Behandlungseinheit (3) eine Elektrolyseeinheit (44) zum Ausführen eines elektrolytischen Chloralkaliverfahrens zum Gewinnen von Ätznatron oder Kalilauge umfasst.

15. Vorrichtung nach einem der Ansprüche 8 bis 14, umfassend zwei Dosierventile (51), die stromaufwärts und stromabwärts von der Reaktionskammer (15) positioniert und konfiguriert sind, Material von der Außenseite in den Reaktor (6) oder von dem Reaktor (6) zu der Außenseite zu überführen, ohne dass die Innenseite und die Außenseite des Reaktors (6) sich je in direkter Kommunikation befinden.

## Revendications

1. Procédé (1) de recyclage de polyesters et / ou de polyamides via un procédé de dépolymérisation, comprenant une réaction de dépolymérisation du matériau à traiter avec une solvolyse; le procédé étant **caractérisé en ce que** la réaction de dépolymérisation comprend les étapes de: fournir un réacteur de dépolymérisation micro-ondes (6) s'étendant sensiblement selon un axe (A); alimenter le matériau à traiter et la solvolyse, définissant les réactifs de la réaction de dépolymérisation et constituant respectivement une phase solide et une phase liquide, vers le réacteur (6) via des lignes d'alimentation respectives (9, 10) ; déplacer les réactifs de la réaction de dépolymérisation à travers le réacteur (6) via un système de déplacement à vis d'Archimède (7) qui déplace les réactifs sensiblement le long de l'axe (A) et mélange les réactifs les uns aux autres pour former un mélange réactif hétérogène ; irradier les réactifs, tout en traversant une chambre de réaction (15) du réacteur (6), avec un rayonnement micro-ondes pour effectuer la réaction de dépolymérisation; la chambre de réaction (15) étant définie à l'intérieur d'un boîtier tubulaire (14) du réacteur (6) ayant une paroi latérale (20) transparente au rayonnement micro-ondes et logée à l'intérieur d'une chambre micro-ondes (16), qui est disposée coaxialement et radialement extérieurement à et autour du boîtier (14) de manière à entourer la chambre de réaction (15); les réactifs étant déplacés au moyen d'une vis sans fin (22) qui s'étend selon l'axe (A) à travers la chambre de réaction (15) et tourne autour de l'axe (A).

2. Procédé selon la revendication 1, dans lequel la vis sans fin (22) est faite d'un matériau métallique et est revêtue d'un matériau à faible permittivité diélectrique ou en PTFE ou matériau céramique.

3. Procédé selon la revendication 1 ou 2, dans lequel la paroi latérale (20) du boîtier (14) est en PTFE, ou en matériau céramique transparent au rayonnement micro-ondes, ou en verre revêtu intérieurement d'un revêtement anticorrosion transparent au rayonnement micro-ondes.

4. Procédé selon la revendication 3, dans lequel le revêtement anticorrosion est constitué d'oxydes, nitrures, carbures ou siliciures de métaux, métaux de transition, lanthanides, actinides ou leurs mélanges de composés.

5. Procédé selon l'une des revendications précédentes, dans lequel la solvolyse comprend de l'éthylène glycol et au moins un hydroxyde alcalin, par exemple NaOH, KOH, LiOH.

6. Procédé selon l'une des revendications précédentes, comprenant un procédé électrolytique au chlore-alcali pour récupérer la soude caustique ou la potasse caustique.

7. Procédé selon l'une des revendications précédentes, comprenant les étapes consistant à alimenter le réacteur (6) en matériau polymérique à traiter et à retirer du réacteur (6) les produits de réaction et les matériaux n'ayant pas réagi, au moyen de valves de dosage respectives (5) afin de transférer du matériau de l'extérieur vers le réacteur (6) et du réacteur (6) vers l'extérieur sans que l'intérieur et l'extérieur du réacteur (6) soient en communication directe.

8. Appareil (1) pour le recyclage de polyesters et / ou de polyamides via un procédé de dépolymérisation, comprenant une unité de dépolymérisation (2) ayant au moins un réacteur de dépolymérisation (6), dans lequel se produit une réaction de dépolymérisation du matériau à traiter avec une solvolyse, et une unité de traitement (3) pour traiter les produits sortant de l'unité de dépolymérisation (2); l'appareil comprenant des lignes d'alimentation respectives (9, 10) connectées au réacteur (6) pour alimenter le réacteur (6) avec le matériau à traiter et la solvolyse, définissant les réactifs de la réaction de dépolymérisation et constituant respectivement une phase solide et une phase liquide ; l'appareil (1) étant **caractérisé en ce que** le réacteur (6) est un réacteur de dépolymérisation à micro-ondes qui s'étend sensiblement selon un axe (A) et est pourvu d'un système mobile (7) des réactifs qui permet au réacteur (6) de fonctionner en continu; le système mobile (7) étant un système à vis d'Archimède qui déplace les réactifs sensiblement le long de l'axe (A) à travers le réacteur (6) et mélange les réactifs pour former un mélange de réaction hétérogène; le réacteur (6) comprenant un boîtier tubulaire (14), qui s'étend le long et autour de l'axe (A) et a une paroi latérale (20) transparente au rayonnement micro-ondes et définissant intérieurement une chambre de réaction (15); le boîtier (14) étant logé à l'intérieur d'une chambre à micro-ondes (16) qui est disposée coaxialement et radialement à l'extérieur du boîtier (14) et autour de celui-ci, de manière à entourer la chambre de réaction (15), et en étant pourvu d'un dispositif générateur de micro-ondes (17); le système mobile (7) comprenant un conduit d'alimentation tubulaire (21), coaxial et sensiblement aligné avec le boîtier (14) selon l'axe (A), et une vis sans fin (22) qui s'étend selon l'axe (A) à l'intérieur du conduit d'alimentation (21) et du boîtier (14) et tournant autour de l'axe (A).

9. Appareil selon la revendication 8, dans lequel la vis sans fin (22) est faite d'un matériau métallique et est revêtue d'un matériau à faible permittivité diélectrique ou de PTFE ou d'un matériau céramique.

10. Appareil selon la revendication 8 ou 9, dans lequel la paroi latérale (20) du boîtier (14) est en PTFE, ou en matériau céramique transparent au rayonnement micro-ondes, ou en verre revêtu intérieurement par un revêtement anticorrosion transparent au rayonnement micro-ondes.

11. Appareil selon la revendication 10, dans lequel le revêtement anticorrosion est constitué d'oxydes, nitrures, carbures ou siliciures de métaux, métaux de transition, lanthanides, actinides ou leurs mélanges de composés.

12. Appareil selon l'une des revendications 8 à 11, dans lequel la solvolyse comprend de l'éthylène glycol et au moins un hydroxyde alcalin, par exemple NaOH, KOH, LiOH.

13. Appareil selon l'une des revendications 8 à 12, et comprenant une pluralité de réacteurs (6) fonctionnant en parallèle et disposés radialement autour d'un collecteur commun (36).

14. Appareil selon l'une des revendications 8 à 13, dans lequel l'unité de traitement (3) comprend une unité d'électrolyse (44) pour exécuter un procédé électrolytique au chlore-alcali pour récupérer de la soude caustique ou de la potasse caustique.

15. Appareil selon l'une des revendications 8 à 14, comprenant deux vannes de dosage (51), positionnées en amont et en aval de la chambre de réaction (15) et configurées pour transférer du matériau de l'extérieur dans le réacteur (6) ou du réacteur (6) vers l'extérieur sans que l'intérieur et l'extérieur du réacteur (6) ne soient jamais en communication directe.
